# EUROPÄISCHE PATENTANMELDUNG

(11) **EP 2 226 022 A1**
(43) Veröffentlichungstag der Anmeldung: **08.09.2010**
(21) Anmeldenummer: 09450049.3
(22) Anmeldetag: 04.03.2009
(51) Int. Cl.: A61B 17/34, A61B 19/00

(54) **Doppelkanüle für die Regionalanästhesie**

(71) Anmelder: Steinbrenner, Marko, 1040 Echallens (CH); Austria Wirtschaftsservice Gesellschaft mbH, 1030 Wien (AT)
(72) Erfinder: Steinbrenner, Marko, Dr. med., 1150 Wien (AT)
(74) Vertreter: Haffner und Keschmann Patentanwälte OG

(57) **Zusammenfassung**

Bei einer Doppelkanüle (1,9) zur Abgabe von Arzneimitteln in der Nähe von Nerven, umfassend eine erste Kanüle (2) mit einem ein spitzes, perforierendes Ende ausbildenden Schliff mit einer Schlifffläche und eine zweite Kanüle (3) mit einem stumpfen Ende, ist die zweite Kanüle (3) zwischen einer ersten, relativ zur ersten Kanüle (2) zurückgezogenen Position und einer zweiten, relativ zur ersten Kanüle (2) vorgeschobenen Position in der ersten Kanüle (2) verschieblich geführt, wobei der am weitesten distal liegende Umfangsabschnitt des Schliffs der ersten Kanüle (2) in der zweiten, relativ zur ersten Kanüle (2) vorgeschobenen Position an der Außenfläche der zweiten Kanüle (3) anliegt oder vom distalen Endbereich der zweiten Kanüle (3) abgedeckt ist. (Fig. 5)

## Beschreibung

Die Erfindung betrifft eine Doppelkanüle zur Abgabe von Arzneimitteln in der Nähe von Nerven, umfassend eine erste Kanüle mit einem ein spitzes, perforierendes Ende ausbildenden Schliff mit einer Schlifffläche und eine zweite Kanüle mit einem stumpfen Ende, wobei die zweite Kanüle zwischen einer ersten, relativ zur ersten Kanüle zurückgezogenen Position und einer zweiten, relativ zur ersten Kanüle vorgeschobenen Position in der ersten Kanüle verschieblich geführt ist.

Neben der Allgemeinanästhesie existieren verschiedene Verfahren der Regionalanästhesie. Während bei der Allgemeinanästhesie Arzneistoffe systemisch verabreicht werden, erfolgt die für den Organismus wesentlich schonendere Regionalanästhesie in der Form, dass entsprechende Arzneimittel entweder rückenmarksnah, wie bei der Epiduralanästhesie oder der Spinalanästhesie oder aber direkt an einen peripheren Nerv verabreicht werden, sodass die Reizleitung aus dem entsprechenden Körperteil unterbunden wird. Zur letztgenannten Form der Regionalanästhesie, der Blockade peripherer Nerven, ist es notwendig, ein Lokalanästhetikum dicht an den betreffenden Nerv zu spritzen, wobei hierfür spezielle Nadeln und Katheter verwendet werden. Entscheidend für eine wirkungsvolle Anästhesie ist hierbei, das Lokalanästhetikum möglichst nahe an den Nerv zu bringen. Die Position der Kanüle relativ zum Nerv wird auf verschiedene Weise kontrolliert, wobei insbesondere Ultraschalltechniken sowie die Elektrostimulation des zu anästhesierenden Nervs Verwendung finden. Große Sorgfalt muss beim Setzen der Kanüle darauf gelegt werden, den Nerv mit der scharfen Kanülenspitze nicht zu verletzen, wobei eine optimale Annäherung an den Nerv nicht immer sofort gelingt, sodass die Kanüle mehrmals zurückgezogen werden muss, um sie erneut in die Nähe des Nervs zu bringen.

Um Verletzungen der Dura beim Setzen einer definitionsgemäß rückenmarksnahen Epiduralanästhesie zu verhindern, ist beispielsweise aus der US 5,685,852 eine Doppelkanüle bekannt geworden, mit welcher Katheter für die Epiduralanästhesie gesetzt werden können. Bei dieser Doppelkanüle ist in einer äußeren spitzen Nadel eine innere stumpfe Nadel geführt, sodass ein in der inneren Nadel geführter Katheter ohne die Gefahr einer Beschädigung von empfindlichem Gewebe eingebracht werden kann.

Um das Verletzungsrisiko des Nervs durch die derzeit in der klinischen Praxis verwendeten, relativ spitzen Spezialnadeln zur peripheren Nervenblockade zu verringern, wäre die Verwendung solcher Doppelkanülen nicht nur zur Epiduralanästhesie sondern für die Regionalanästhesie peripherer Nerven prinzipiell vorteilhaft. Es hat sich hierbei jedoch beim Zurückziehen und Vorschieben der Kanüle im Gewebe ein gewisser hobelnder Effekt negativ bemerkbar gemacht, welcher daher rührt, dass sich zumindest in einem distalen Teilbereich des Schliffs der äußeren scharfen Kanüle zwischen der inneren und der äußeren Kanüle ein Spalt befindet. Gewebe, das beim Vorschieben in diesen Spalt gerät, wird durch die scharfe äußere Kanüle abgeschnitten bzw. ausgestanzt, was einerseits zu einer schlechten Kontrollierbarkeit der Kanüle und andererseits zu teilweise erheblichen Gewebsschädigungen führt.

Es ist somit Aufgabe der vorliegenden Erfindung, eine Doppelkanüle der eingangs genannten Art für die Blockade peripherer Nerven besser anzupassen und insbesondere dahingehend zu verbessern, dass ein Stanzen bzw. Hobeln des Gewebes beim Vorschieben der Kanüle an der Spitze zwischen der äußeren Kanüle und der inneren Kanüle vermieden wird. Zur Lösung dieser Aufgabe ist eine Doppelkanüle der eingangs genannten Art erfindungsgemäß dahingehend weitergebildet, dass der am weitesten distal liegende Umfangsabschnitt des Schliffs der ersten Kanüle in der zweiten, relativ zur ersten vorgeschobenen Position an der Außenfläche der zweiten Kanüle anliegt oder vom distalen Endbereich der zweiten Kanüle abgedeckt ist. Dadurch, dass der am weitesten distal liegende Umfangsabschnitt des Schliffs der ersten Kanüle in der zweiten, relativ zur ersten vorgeschobenen Position an der Außenfläche der zweiten Kanüle anliegt oder abgedeckt ist, wird ein Spalt zwischen der äußeren und der inneren Kanüle vermieden oder abgedeckt, wodurch auch bei mehrmaligem Vor- und Zurückschieben der Doppelkanüle im Gewebe eine Hobelwirkung vermieden wird.

Um die erfindungsgemäß vorgesehene Anlage des am weitesten distal liegenden Umfangsabschnitts des Schliffs der ersten Kanüle an der Außenfläche der zweiten Kanüle zu gewährleisten, ist die Erfindung mit Vorteil dahingehend weitergebildet, dass die zweite Kanüle als Knopfkanüle ausgebildet ist. Bei einer solchen Ausbildung kann die Spitze der ersten Kanüle mit einem herkömmlichen Einfachschliff oder einem ähnlichen schrägen Schliff ausgeführt sein, wobei der problematische distal gelegene Bereich der Schlifffläche, welcher aufgrund der Neigung der Schlifffläche gemeinsam mit der Außenfläche der zweiten Kanüle den Spalt ausbildet, durch den proximalen Bereich des Knopfes der Knopfkanüle abgedeckt ist. Somit kann kein Gewebe eingeklemmt werden und das Zurück- und Vorschieben der Kanüle bei der tastenden Annäherung an den Nerv, welche mit der erfindungsgemäßen Doppelkanüle durch das stumpfe Ende der zweiten Kanüle ermöglicht wird, erfolgt besonders gewebeschonend. Ein zusätzlicher Vorteil ergibt sich bei einer solchen Ausführungsform aus der Tatsache, dass Knopfkanülen, welche hier als die zweite Kanüle Verwendung finden, an sich besonders gewebeschonend sind und aufgrund ihrer Geometrie von festem Gewebe abgelenkt werden. Ein zu blockierender Nerv lenkt die Kanüle beim Platzieren aufgrund seiner relativen Festigkeit ab, sodass dieser Effekt die korrekte Platzierung der Doppelkanüle erleichtert. Dadurch, dass in der zweiten relativ zur ersten Kanüle vorgeschobenen Position eine Knopfkanüle an der Spitze der Doppelkanüle zu liegen kommt und gleichzeitig der distal gelegene Bereich der Schlifffläche durch die Knopfkanüle abgedeckt ist, können anatomische Strukturen, die das weitere Vorschieben der Nadel behindern würden, deplatziert werden. Dies können andere Nerven aber auch Blutgefäße sein, welche den Weg der Nadelspitze von der Einstichstelle der Kanüle zum zu blockierenden Nerv behindern und deren Manipulation zur Deplatzierung mit einer scharfen Nadel ein erhebliches Verletzungsrisiko beinhalten würde. Mit der gewebeschonenden Spitze einer Knopfkanüle und dem abgedeckten kritischen distal gelegenen Bereich der Schlifffläche können solche Strukturen nach dem Einstich der Doppelkanüle ohne großes Risiko zur Seite gedrängt werden, sodass ein weiterer Einstich, um diese Hindernisse zu umgehen, entfallen kann.

Die Doppelkanüle kann hierbei bevorzugt dahingehend weitergebildet sein, dass der distale Bereich der Spitze der ersten Kanüle in Richtung des Auges der ersten Kanüle gebogen ist. Bei einer solchen Ausführungsform läuft die Knopfkanüle beim Verschieben in die zweite, relativ zur ersten Kanüle vorgeschobenen Position auf den gebogenen Bereich der Spitze der ersten Kanüle auf, sodass für den Operateur ein Widerstand spürbar wird, aus dem geschlossen werden kann, dass das Austreten der zweiten Kanüle aus der Spitze der ersten Kanüle unmittelbar bevorsteht. Wenn nun die zweite Kanüle gegen diesen Widerstand weiter vorgeschoben wird, weicht der gebogene distale Bereich der Spitze der ersten Kanüle dem Knopf der zweiten Kanüle elastisch aus und gelangt hinter dem größten Durchmesser des Knopfes der zweiten Kanüle zur Anlage an die Außenfläche der zweiten Kanüle. In diesem Zustand ist der distale Bereich der Spitze vollständig hinter dem Knopf der zweiten Kanüle verborgen, sodass kein hobelnder Spalt gebildet werden kann.

In bevorzugter Weise kann die Anlage des am weitesten distal liegenden Umfangsabschnitts des Schliffs der ersten Kanüle an der Außenfläche der zweiten Kanüle auch dadurch bewirkt werden, dass die Schlifffläche der ersten Kanüle pfeilförmig von der Spitze weg geneigt ist. Durch einen solchen Schliff liegt der am weitesten distal liegende Umfangsabschnitt des Schliffs der ersten Kanüle am Innenumfang der Kanüle vor und kann somit mit der Außenfläche der zweiten Kanüle in Anlage kommen. Auch auf diese Weise wird ein Spalt zwischen der ersten und der zweiten Kanüle vermieden.

In besonders bevorzugter Weise ist die Doppelkanüle dahingehend weitergebildet, dass die Schlifffläche der ersten Kanüle pfeilförmig von der Spitze weggeneigt ist und die zweite Kanüle als Knopfkanüle ausgebildet ist. Eine solche Ausführungsform stellt eine Kombination des besonderen Schliffs der ersten Kanüle und der Ausbildung der zweiten Kanüle als Knopfkanüle dar, sodass eine optimale Gewebeschonung erreicht wird.

Um nach dem erfolgreichen Positionieren der Kanüle und insbesondere des stumpfen Endes der zweiten Kanüle in der Nähe des zu anästhesierenden Nervs ein entsprechendes Anästhetikum abgeben zu können, ist die Doppelkanüle mit Vorteil dahingehend weitergebildet, dass eine Zuspritzeinrichtung für Arzneimittel an der zweiten Kanüle festlegbar ist. Eine solche Zuspritzeinrichtung kann in Form einer einfachen Spritze ausgeführt sein, wobei jedoch auch Ausführungsformen denkbar sind, bei denen die Zuspritzeinrichtung von einer automatischen Dosierapparatur gebildet ist.

In bevorzugter Weise ist die Doppelkanüle dahingehend weitergebildet, dass die Zuspritzeinrichtung für Arzneimittel als Zuspritzschlauch ausgebildet ist. Ein solcher Zuspritzschlauch fungiert als Abstandhalter zwischen der Doppelkanüle und einer Spritze, die an den Zuspritzschlauch angesetzt werden kann. Durch das Vorsehen eines Zuspritzschlauches als Abstandhalter, wird vermieden, dass die in der Nähe des zu blockierenden Nervs platzierte Doppelkanüle durch das Ansetzen der Spritze verrutscht. Das Ansetzen der Spritze wird somit bedeutend erleichtert und kann in der Folge auch vom OP-Personal durchgeführt werden.

Um die Doppelkanüle und insbesondere die zweite Kanüle in der relativ zur ersten Kanüle vorgeschobenen Position exakt führen zu können, ist die Erfindung gemäß einer bevorzugten Ausführungsform dahingehend weitergebildet, dass die zweite Kanüle aus einem hoch biegefesten Material gefertigt ist.

In bevorzugter Weise ist die erfindungsgemäße Doppelkanüle dahingehend weitergebildet, dass zumindest eine der beiden Kanülen eine echogene bzw. radiopaque Beschichtung aufweist. Eine solche echogene bzw. radiopaque Beschichtung verbessert die Sichtbarkeit der Doppelkanüle in bildgebenden Verfahren, wie Ultraschall oder Röntgen, und kann somit eine visuelle Kontrolle der Annäherung der Doppelkanüle an den zu blockierenden Nerv verbessern.

Wie bereits eingangs erwähnt, kann zur Kontrolle der Annäherung an den Nerv das Verfahren der Elektrostimulation des Nervs Verwendung finden. Um eine solche Elektrostimulation zu ermöglichen, ist die Doppelkanüle bevorzugt dahingehend weitergebildet, dass die Doppelkanüle (1,9) Anschlüsse für eine Elektrostimulationsvorrichtung und der Schaft zumindest der mit der Elektrostimulationsvorrichtung verbundenen Kanüle eine elektrische Isolationsschicht aufweist. Über diese Anschlüsse kann ein Gleichstrom über die Kanüle geführt werden, sodass dann, wenn die Kanülenspitze in der Nähe des Nervs positioniert ist, eine entsprechende Muskelkontraktion beobachtet werden kann. Der Schaft der Doppelkanüle ist hierbei naturgemäß derart isoliert, dass der Stromeintritt in den Körper nur über die Kanülenspitze erfolgt, um die Annäherung der Spitze der Doppelkanüle an den Nerv beurteilen zu können.

Mit Vorteil ist die Doppelkanüle gemäß der vorliegenden Erfindung dahingehend weitergebildet, dass der innere Durchmesser der zweiten Kanüle dem Durchmesser eines Anästhesiekatheters entspricht, sodass nach der Annäherung der Kanüle an den Nerv ein Katheter eingebracht werden kann, der längerfristig im Körper verbleiben kann, was eine kontinuierliche bzw. intermittierende Anästhesie, eine postoperative Analgesie aber auch die Therapie chronischer Schmerzzustände ermöglicht.

Um das Einführen eines Anästhesiekatheters in die zweite Kanüle zu erleichtern, ist die Doppelkanüle mit Vorteil dahingehend weitergebildet, dass am proximalen Ende der zweiten Kanüle eine Einführvorrichtung für einen Anästhesiekatheter festgelegt ist.

Mit Vorteil ist die Erfindung dahingehend weitergebildet, dass die beiden Nadeln zumindest in der ersten, relativ zur ersten Kanüle zurückgezogenen Position und in der zweiten, relativ zur ersten Kanüle vorgeschobenen Position der zweiten Kanüle relativ zueinander arretierbar sind. Dadurch, dass die beiden Nadeln zumindest in der ersten, relativ zur ersten Kanüle zurückgezogenen Position und in der zweiten, relativ zur ersten Kanüle vorgeschobenen Position der zweiten Kanüle relativ zueinander arretierbar sind, ist sichergestellt, dass die beiden funktionellen Positionen, nämlich die erste Position zum Durchstechen der Haut und zum Einbringen der Kanüle in die Umgebung des Nerven, sowie die zweite Position zum gewebeschonenden Herantasten an den Nerv jeweils gesichert werden können und unbeabsichtigte und unbemerkte Veränderungen der Charakteristik der Doppelkanüle in den jeweiligen Positionen sicher ausgeschlossen werden können.

Um dem Operateur jederzeit einen Eindruck zu vermitteln, in welcher Tiefe sich die Doppelkanüle im Körper befindet, ist die Erfindung mit Vorteil dahingehend weitergebildet, dass an der Außenseite der ersten Kanüle eine Markierung zum Bestimmen der Eindringtiefe der Doppelkanüle angeordnet ist.

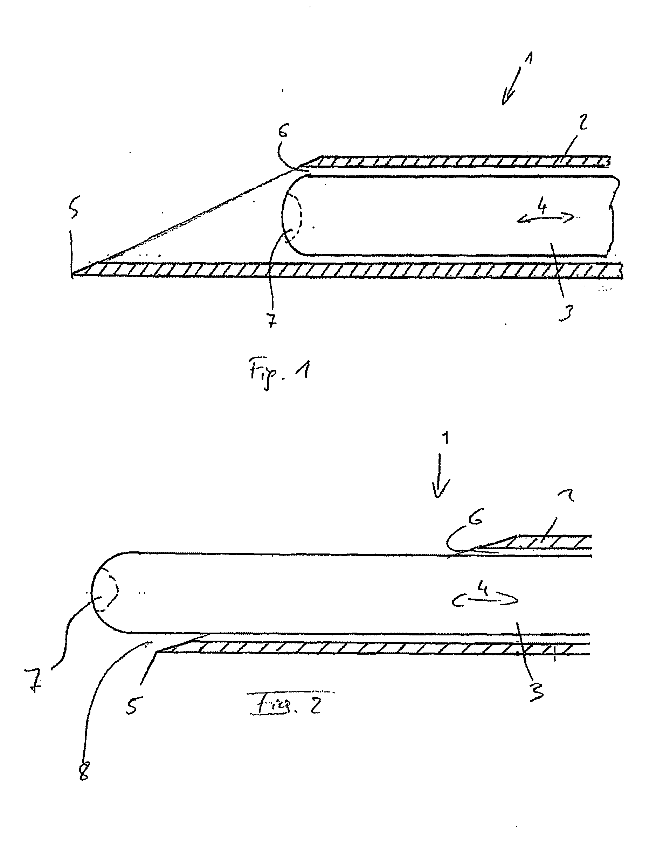

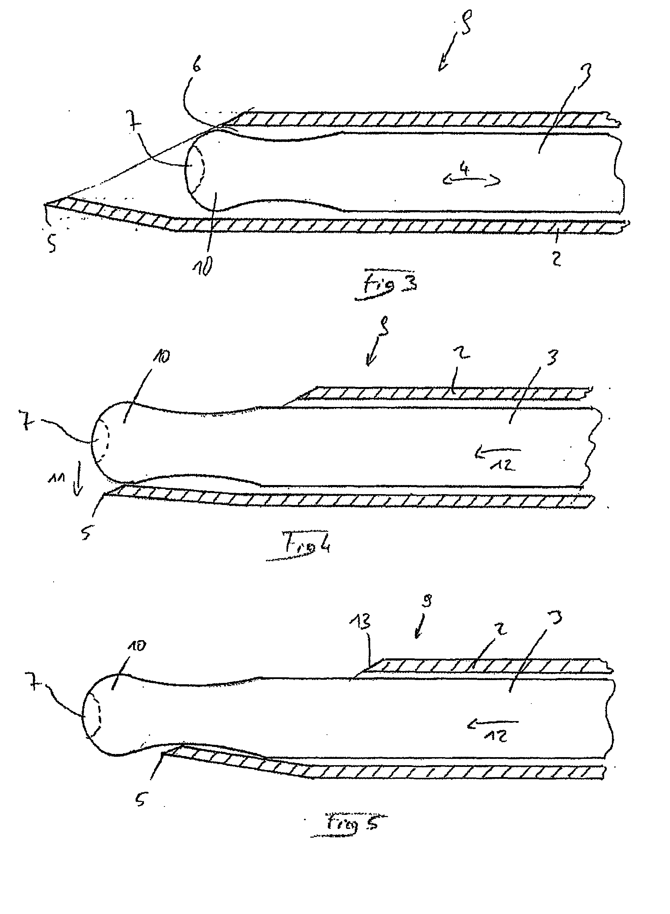

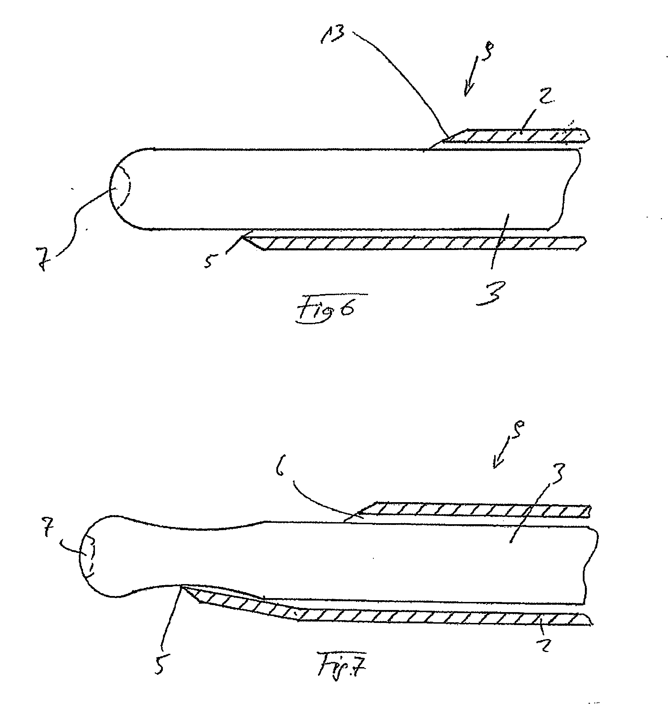

Die Erfindung wird nachfolgend anhand eines in der Zeichnung schematisch dargestellten Ausführungsbeispiels näher erläutert. In dieser zeigen die Figuren 1 und 2 eine Doppelkanüle aus dem Stand der Technik, die Figur 3 eine Ausführungsform der vorliegenden Erfindung, bei welcher die zweite Kanüle als Knopfkanüle ausgeführt ist und bei welcher die Spitze der ersten Kanüle in Richtung des Auges ersten Kanüle gebogen ist, in einer ersten, relativ zur ersten Kanüle zurückgezogenen Position der zweiten Kanüle, Figur 4 die erfindungsgemäße Doppelkanüle gemäß Figur 3 in einer Position zwischen der ersten, relativ zur ersten Kanüle zurückgezogenen Position und der zweiten, relativ zur ersten Kanüle vorgeschobenen Position, Figur 5 eine Darstellung der Doppelkanüle gemäß den Figuren 3 und 4, bei welcher sich die zweite Kanüle in der zweiten, relativ zur ersten Kanüle vorgeschobenen Position befindet, Figur 6 eine Ausführungsform der vorliegenden Erfindung, bei welcher die Schlifffläche der ersten Kanüle pfeilförmig von der Spitze weggeneigt ist, bei welcher sich die zweite Kanüle in der zweiten, relativ zur ersten Kanüle vorgeschobenen Position befindet und Figur 7 eine erfindungsgemäße Doppelkanüle, bei welcher die erste Kanüle einen pfeilförmigen Schliff aufweist und die zweite Kanüle als Knopfkanüle ausgebildet ist, wobei sich die zweite Kanüle ebenfalls in der zweiten, relativ zur ersten Kanüle vorgeschobenen Position befindet.

In Fig. 1 ist mit 1 eine Doppelkanüle bezeichnet, welche aus einer ersten Kanüle 2 mit einem ein spitzes, perforierendes Ende ausbildenden Schliff und einer zweite Kanüle 3 mit einem stumpfen Ende besteht. Die zweite Kanüle 3 ist in Richtung des Doppelpfeils 4 in der ersten Kanüle 2 verschieblich geführt und kann über das distale Ende 5 der Kanüle 2 hinaus verschoben werden. Mit 6 ist das Auge bzw. die Öffnung der ersten Kanüle 2 und mit 7 das Auge bzw. die Öffnung der zweiten Kanüle 3 bezeichnet. In Fig. 2 ist erkennbar, dass dann wenn die zweite Kanüle 3 über das distale Ende 5 der Kanüle 2 hinaus verschoben ist, ein Spalt 8 zwischen dem am weitesten distal liegenden Umfangsabschnitt der Schlifffläche der ersten Kanüle 2 und der Außenfläche der zweiten Kanüle 3 gebildet wird. Wenn nun eine solche Doppelkanüle in die Nähe eines zu blockierenden Nervs bei der Regionalanästhesie gebracht werden soll und es dabei erforderlich ist, die Nadel mehrmals vor- und zurückzubewegen, so kann Gewebe in den Spalt 8 eindringen und in der Folge abgehobelt, bzw. ausgestanzt werden.

In Fig. 3 ist nun eine erfindungsgemäße Doppelkanüle mit 9 bezeichnet, wobei entsprechende Bauteile mit gleichen Bezugszeichen, wie in den Figuren 1 und 2 bezeichnet wurden. In Fig. 3, ist die zweite Kanüle 3 als Knopfkanüle ausgebildet und ebenfalls entlang des Doppelpfeils 4 in der ersten Kanüle 2 verschieblich geführt. Das distale Ende 5 der ersten Kanüle 2 ist hierbei in Richtung des Auges 6 der ersten Kanüle 2 gebogen, sodass der kugelförmige Kopf 10 der zweiten Kanüle 3 auf die innere Mantelfläche der ersten Kanüle 2 aufläuft.

In Fig. 4 ist zu erkennen, dass in einer weiter vorgeschobenen Position der zweiten Kanüle 3 das distale Ende 5 der Spitze in Richtung des Pfeils 11 elastisch gebogen wird, wodurch der Operateur einen Widerstand verspürt, der es ihm ermöglicht, die relative Position der beiden Kanülen 2 und 3 zueinander zu erfühlen. Bei einer weiteren Verschiebung der zweiten Kanüle 3 in Richtung des Pfeils 12 (Fig. 5) federt das distale Ende 5 der Spitze der ersten Kanüle 2 zurück und der kugelförmige Kopf 10 der zweiten Kanüle 3 deckt den distalen Bereich des Schliffs der Spitze 5 der ersten Kanüle 2 ab, sodass kein Gewebe in einen Spalt zwischen der ersten Kanüle 2 und der zweiten Kanüle 3 eindringen und in der Folge abgehobelt werden kann. Das proximale Ende 13 der Spitze der ersten Kanüle 2, welches bei diesem Ausführungsbeispiel als Einfachschliff ausgeführt ist, weist mit seiner Schlifffläche pfeilförmig nach hinten, sodass der proximal liegende Umfangsabschnitt des Schliffs in diesem Bereich ebenfalls an der Außenfläche der zweiten Kanüle anliegt und auch in diesem Bereich kein Spalt vorliegt.

In Fig. 6 ist eine weitere Ausführungsform der vorliegenden Erfindung dargestellt, bei welcher entsprechende Bauteile wiederum mit gleichen Bezugszeichen versehen sind. In Fig. 6 ist erkennbar, dass die zweite Kanüle 3 ebenfalls ein stumpfes Ende und ein zentrales Auge 7 aufweist, wobei jedoch die zweite Kanüle 3 nicht als Knopfkanüle ausgebildet ist. Die Spitze 5 der ersten Kanüle muss folglich einen speziellen Schliff aufweisen, damit der am weitesten distal liegende Umfangsabschnitt des Schliffs ebenfalls an der ersten Kanüle 2 anliegt. Wie bei einem Einfachschliff, ist das proximale Ende 13 pfeilförmig nach hinten geneigt. Zum Unterschied zu einem Einfachschliff, ist der Schliff jedoch so ausgeführt, dass die Schlifffläche auch am distalen Ende der Spitze 5 pfeilförmig nach hinten geneigt ist, sodass wiederum der am weitesten distal liegende Umfangsabschnitt des Schliffs der ersten Kanüle 2 in der zweiten, relativ zur ersten Kanüle vorgeschobenen Position der zweiten Kanüle 3 an der Außenfläche der zweiten Kanüle anliegt. Auch bei einer solchen Ausbildung der Doppelkanüle gemäß der vorliegenden Erfindung wird somit im wesentlichen kein Spalt gebildet, sodass auch hier kein Gewebe ausgestanzt werden kann.

In Fig. 7 ist nun eine Kombination der Ausführungsform, bei welcher die zweite Kanüle in Form einer Knopfkanüle ausgebildet ist und der Ausführungsform bei welcher die erste Kanüle einen Schliff aufweist, bei welchem die Schlifffläche auch am distalen Ende der Spitze 5 pfeilförmig nach hinten geneigt ist, dargestellt. Zusätzlich ist erkennbar, dass das distale Ende der Spitze 5 zum Auge 6 der ersten Kanüle gebogen ist, sodass einerseits der oben beschriebene Widerstand für den Operateur spürbar ist und andererseits eine verbesserte und glattere Anlage der Spitze 5 der ersten Kanüle 2 an die Außenfläche der zweiten Kanüle 3 gewährleistet ist.

## Patentansprüche

1. Doppelkanüle zur Abgabe von Arzneimitteln in der Nähe von Nerven, umfassend eine erste Kanüle mit einem ein spitzes, perforierendes Ende ausbildenden Schliff mit einer Schlifffläche und eine zweite Kanüle mit einem stumpfen Ende, wobei die zweite Kanüle zwischen einer ersten, relativ zur ersten Kanüle zurückgezogenen Position und einer zweiten, relativ zur ersten Kanüle vorgeschobenen Position in der ersten Kanüle verschieblich geführt ist, **dadurch gekennzeichnet, dass** der am weitesten distal liegende Umfangsabschnitt des Schliffs der ersten Kanüle (2) in der zweiten, relativ zur ersten Kanüle (2) vorgeschobenen Position an der Außenfläche der zweiten Kanüle (3) anliegt oder vom distalen Endbereich der zweiten Kanüle (3) abgedeckt ist.

2. Doppelkanüle nach Anspruch 1, **dadurch gekennzeichnet, dass** die zweite Kanüle (3) als Knopfkanüle ausgebildet ist.

3. Doppelkanüle nach Anspruch 1 oder 2, **dadurch gekennzeichnet, dass** der distale Bereich der Spitze (5) der ersten Kanüle (2) in Richtung des Auges (6) der ersten Kanüle (2) gebogen ist.

4. Doppelkanüle nach einem der Ansprüche 1, 2 oder 3, **dadurch gekennzeichnet, dass** die Schlifffläche der ersten Kanüle (2) pfeilförmig von der Spitze (5) weg geneigt ist.

5. Doppelkanüle nach einem der Ansprüche 1 bis 4, **dadurch gekennzeichnet, dass** die Schlifffläche der ersten Kanüle (2) pfeilförmig von der Spitze (5) weggeneigt ist und die zweite Kanüle (3) als Knopfkanüle ausgebildet ist.

6. Doppelkanüle nach einem der Ansprüche 1 bis 5, **dadurch gekennzeichnet, dass** eine Zuspritzeinrichtung für Arzneimittel an der zweiten Kanüle (3) festlegbar ist.

7. Doppelkanüle nach einem der Ansprüche 1 bis 6, **dadurch gekennzeichnet, dass** die Zuspritzeinrichtung für Arzneimittel als Zuspritzschlauch ausgebildet ist.

8. Doppelkanüle nach einem der Ansprüche 1 bis 7, **dadurch gekennzeichnet, dass** die zweite Kanüle (3) aus einem hoch biegefesten Material gefertigt ist.

9. Doppelkanüle nach einem der Ansprüche 1 bis 8, **dadurch gekennzeichnet, dass** zumindest eine der beiden Kanülen (2,3) eine echogene bzw. radiopaque Beschichtung aufweist.

10. Doppelkanüle nach einem der Ansprüche 1 bis 9, **dadurch gekennzeichnet, dass** die Doppelkanüle (1,9) Anschlüsse für eine Elektrostimulationsvorrichtung und der Schaft zumindest der mit der Elektrostimulationsvorrichtung verbundenen Kanüle eine elektrische Isolationsschicht aufweist.

11. Doppelkanüle nach einem der Ansprüche 1 bis 10, **dadurch gekennzeichnet, dass** der innere Durchmesser der zweiten Kanüle (3) dem Durchmesser eines Anästhesiekatheters entspricht.

12. Doppelkanüle nach einem der Ansprüche 1 bis 11, **dadurch gekennzeichnet, dass** am proximalen Ende der zweiten Kanüle (3) eine Einführvorrichtung für einen Anästhesiekatheter festgelegt ist.

13. Doppelkanüle nach einem der Ansprüche 1 bis 12, **dadurch gekennzeichnet, dass** die beiden Kanülen (2,3) zumindest in der ersten, relativ zur ersten Kanüle (2) zurückgezogenen Position und in der zweiten, relativ zur ersten Kanüle (2) vorgeschobenen Position der zweiten Kanüle (3) relativ zueinander arretierbar sind.

14. Doppelkanüle nach einem der Ansprüche 1 bis 13, **dadurch gekennzeichnet, dass** an der Außenseite der ersten Kanüle (2) eine Markierung zum Bestimmen der Eindringtiefe der Doppelkanüle angeordnet ist.
